(19) Europäisches Patentamt
European Patent Office
Office européen des brevets

(11) EP 0 517 338 B1

(12) **EUROPEAN PATENT SPECIFICATION**

(45) Date of publication and mention of the grant of the patent:
**05.03.1997 Bulletin 1997/10**

(51) Int Cl.6: **G01N 33/543**, G01N 33/68, G01N 33/78, G01N 33/94 // G01N33/58

(21) Application number: **92201625.8**

(22) Date of filing: **04.06.1992**

(54) **Analytical element coated by a gravure process**

Analytisches Element beschichtet durch ein Ätzverfahren

Elément d'analyse revêtu par un procédé de gravure

(84) Designated Contracting States:
**AT BE CH DE DK ES FR GB GR IT LI LU MC NL PT SE**

(30) Priority: **07.06.1991 US 713239**

(43) Date of publication of application:
**09.12.1992 Bulletin 1992/50**

(73) Proprietor: **Johnson & Johnson Clinical Diagnostics, Inc.**
**Rochester New York 14650 (US)**

(72) Inventors:
- **Dappen, Glen M.,**
  **c/o EASTMAN KODAK COMPANY**
  **Rochester, New York 14650-2201 (US)**
- **Hassett, James W.,**
  **c/o EASTMAN KODAK COMPANY**
  **Rochester, New York 14650-2201 (US)**
- **Heinle, James F.,**
  **c/o EASTMAN KODAK COMPANY**
  **Rochester, New York 14650-2201 (US)**

(74) Representative: **Mercer, Christopher Paul et al**
**Carpmaels & Ransford**
**43, Bloomsbury Square**
**London WC1A 2RA (GB)**

(56) References cited:
**EP-A- 0 013 156**
**EP-A- 0 209 378**
**EP-A- 0 277 723**
**WO-A-82/02601**
**US-A- 4 461 829**

- **DATABASE WPIL Week 9016, Derwent Publications Ltd., London, GB; AN 90-119768**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to clinical chemistry; to an element for immunoassays and to methods for the determination of an immunologically reactive ligand.

Immunoassays, which take advantage of natural immunological reactions, have found wide-spread use as analytical techniques in clinical chemistry. Because of the specificity of the reactions, they are particularly advantageous in quantifying biological analytes that are present in very low concentration in biological fluids. Such analytes include, for example, antibodies, therapeutic drugs, narcotics, enzymes, hormones, proteins, and so forth

The analyte, which is the target of the assay is referred to herein as the ligand, and the labeled analyte is referred to as the labeled ligand (including immunocompetent derivatives and analogs of such ligand). Compounds which specifically recognize the ligand and the labeled ligand and react to form complexes with them are referred to herein as receptors. The receptor and the ligand or labeled ligand form a conjugate pair. Any member of the pair can function as a receptor or a ligand.

In competitive binding immunoassays, a labeled ligand is placed in competition with unlabeled ligand for reaction with a fixed amount of the appropriate receptor. Unknown concentrations of the ligand can be determined from the measured signal of either the bound or unbound (i.e. free) labeled ligand. The reaction proceeds as follows:

$$\text{ligand} + \text{labeled ligand} + \text{receptor} \Leftrightarrow$$

$$\text{ligand-receptor} + \text{labeled ligand-receptor}.$$

Conventional labels include radioactive tags, enzymes, chromophores, fluorophores, stable free radicals, and enzyme cofactors, inhibitors and allosteric effectors.

Immunoassay analytical elements are known from U.S. Patents 4,517,288 and 4,258,001. In general such elements comprise receptors, such as antibodies for a ligand, immobilized in a particulate layer. In addition the element usually contains a reagent system that through interaction with a bound or unbound species results in a signal that can be correlated to the concentration of ligand in a sample. In use the sample is manually combined with an enzyme labeled ligand and applied to the element. After a certain time a solution containing a substrate for the labeled ligand is applied to the particulate layer. The substrate is catalyzed by the enzyme label to form a reaction product that ultimately causes a signal color to develop. The reflection density of the color can be correlated to the concentration of the ligand in the sample.

Frequently the above analytical elements are used in highly automated immunoassays. The need to add labeled ligand to the sample before conducting the assay reduces the potential output of automated systems.

Both U.S. Patents 4,517,288 and 4,258,001 suggest use of a labeled ligand coated over the particulate layer in which the receptor is immobilized. The latter patent teaches using a barrier layer between the coated labeled ligand coating and the particulate layer in order to avoid prereaction between the receptor and the labeled ligand. The problem is that neither of these patents teach one skilled in the art how to make an immunoassay element having a labeled ligand coated directly over the layer containing the immobilized receptor, without an intervening barrier layer, in a way that avoids (a) inactivation of receptors, such as antibodies, and (b) deleterious prebinding between the labeled ligand and the receptor. Such prebinding would prevent completion of an immunoassay within a commercially reasonable time and certainly take greater than 20 minutes. In other words the prior art is not enabling.

EP-A-0 277 723 discloses an assay in which a ligand sample is incubated with a first, solid phase binding reagent and a second, liquid phase, labelled binding reagent, wherein, prior to incubation, the second binding reagent is contained within a layer on which the first binding agent is immobilised.

US-A-4 461 829 discloses an assay element comprising a solid carrier containing the reagents necessary for carrying out a homogeneous specific binding assay.

The objective of the present invention is to overcome the need to add labeled ligand to the sample or element during the actual assay procedure. This objective is accomplished by providing a dry immunoassay analytical element, as defined in claim 1.

The immunoassay element of this invention is substantially free of prebinding in that the dose response curve of an immunoassay carried out on such an element is substantially the same as the dose response curve obtained with the same element except that the labeled ligand is not coated in the element. Instead the labeled ligand is applied to the element, concurrently with the sample to be tested.

Moreover the element does not include a barrier layer between the particulate receptor containing layer and the layer containing the labeled ligand thereby obviating the need for an extra coating step and additional barrier layer materials. This is accomplished without any significant adverse affect on the receptors.

The present invention also provides a method for the assay of an immunologically reactive ligand in a liquid using

the above element, as defined in claim 12.

Figures 1-4 are curves that illustrate the prebinding that occurs between an antibody and labeled ligand when the labeled ligand is coated with the coating processes used in comparative examples 1-4.

Figures 5-6 are curves showing the substantial absence of prebinding using the coating processes of examples 5-6.

The elements of this invention comprise (a) a coating of a labeled ligand over (b) a particulate layer containing immobilized receptors for the ligand. The element can include additional layers such as those described infra. All of such layers, except the labeled ligand coating, can be coated using coating techniques known in this art and which are briefly described infra. However the coatings containing the labeled ligand are gravure coated according to the procedures described infra.

As noted previously the overcoated labeled ligand is substantially free of any binding reactions with the receptor. Prebinding is substantially avoided by careful co-optimization 1) of minimized wet coverage of the labeled ligand coating composition while at the same time maintaining enough wetness to achieve uniform coverage of the labeled ligand and 2) rapid drying in a way that a) removes substantially all of the coating solvent; and b) maintains sufficient enzyme activity.

The relative affinity of antibody and labeled ligand for each other is also an important factor in minimizing prebinding. This factor is controlled, as is well known by those skilled in this art, by manipulating the structure of the labeled ligand together with a prudent choice of antibody.

In general the level of coated labeled ligand coverage needed in an element provided by the present invention is determined empirically for each specific immunoassay according to the method of claim 9. This method generally involves the following procedure:

1. Determine the concentration of labeled ligand needed to achieve acceptable immunoassay performance when the immunoassay is performed by contacting the analytical element with the labeled ligand concurrently with a sample. Acceptable assay performance is achieved when (a) the assay can be carried out in less than 20 minutes; (b) the dynamic range of the assay is such that the minimum and maximum ligand concentrations detectable cover a clinically useful concentration range; and (c) clinically significant ligand concentrations can be detected across the dynamic range.

2. Empirically determine the level of coated labeled ligand coverage needed with the same analytical element to achieve the above established acceptable assay performance by:

   A. Coating, directly over the particulate receptor layer of the element used to establish optimum spotted labeled ligand levels, the labeled ligand at a coverage in g/m$^2$ that is some fraction, multiple or the same as the concentration of labeled ligand used in spotting the labeled ligand in 1, supra.

   B. Conduct a series of assays with test samples containing a known concentration of the ligand.

   C. Compare the results of the assays with the known concentration of ligand; and

   D. Repeat steps B and C as needed, varying the labeled ligand coverage according to the results seen in step 2C to determine the labeled ligand coverage required.

Depending on the labeled ligand, the coverage of the labeled ligand could be less than, the same or several multiples greater (2X, 3X, 4X, and so forth) than the labeled ligand concentration needed when the same assay is carried out by spotting the labeled ligand directly on the analytical element.

Using the above guidelines, carefully controlled gravure coating procedures were successfully carried out using the following coverages and drying protocols. The labeled ligand coatings in the examples were prepared with a gravure machine ( made by IMD Corporation, Birmingham, Alabama) having two 15 foot arch flotation drying sections. Drying conditions used for all of the examples were 180°F (82°C) in the first drying section only. The second section was not used. The air flow in the dryer was adjusted to maintain adequate flotation throughout the length of the dryer such that the coatings were not scratched from either the support side or the coated side as they were transported through the dryer. The gravure cylinder used contained 295 cells/inch$^2$ (1.344 x 10$^{-8}$ cells/m$^2$). The cells had a depth of 19 μm, a width of 72 μm and a land width between cells of 12 μm. This cylinder will deliver 4.3 g/m$^2$ of coating composition containing the labeled ligand to the bead spreading layer using the direct gravure process at a coating machine speed of 50 ft/min (15.24 m/minute). Those skilled in the gravure coating arts will be readily able to adapt the previously described procedure to any gravure coating machine. The coating composition for the labeled ligand was as follows:

| FIGURE I | |
|---|---|
| Coated Labeled Ligand Coating Composition Based on 4.3 g/m$^2$ Wet Coverage | |
| Component | g/m$^2$ Dry Coverage |
| MOPS Buffer | .0045 |
| BSA (Bovine Serum Albumin) | .000215 |
| A-100 poly-(n-isopropyl acrylamide) | .00108 |
| 4-Hydroxyacetanilide | .000325 |
| *Labeled ligand | .000016 |

*Labeled Ligand has been coated anywhere between 4 and 64 μg/m$^2$

As previously stated the remaining layers of the element can be coated using well known coating techniques in this art. For example slide-extrusion hoppers of the type described in U.S. Patent 2,761,417 are often advantageous for simultaneous coating of a plurality of layers at least one of which is comprised of polymeric particles bearing immobilized beads. More particularly, a multilayer element can be coated by directing a coating composition containing the beads through an extrusion slot of a slide extrusion hopper and simultaneously flowing a layer of a second coating composition, which, if desired, may also contain beads down a slide surface of the slide-extrusion hopper. Preferably, the coating composition flowing through the extrusion slot is supplied at a volumetric flow rate that is substantially greater than the volumetric flow rate of the coating composition flowing down the slide surface. Also, it is desirable that the coating composition directed through the extrusion slot have a viscosity which is substantially higher than the viscosity of the coating composition flowing down the slide surface. It should also have a surface tension which is at least about as high and, most preferably, somewhat higher than that of the composition coating flowing down the slide surface. Control of the coating parameters of flow rate, viscosity and surface tension in this manner aids in promoting the formation of discrete layers that are free from interlayer mixing and in avoiding the formation of repellency defects.

The particulate layer in which the receptors are immobilized is porous. Materials for use in such layers are well known in the art of making dry analytical elements as disclosed, for example, in U.S. Patent 4,258,001. Such layers include macroporous layers made from cloth, paper, and so forth. A preferred particulate layer is a bead spreading layer (BSL). This layer can be easily constructed to have suitable porosity for use in the elements of the present invention to accommodate a test sample (for example 1 to 100 μL), diluted or undiluted. Preferably, the spreading layer is isotropically porous, which property is created by interconnected spaces between the particles comprising the zone. By isotropically porous is meant that the spreading layer uniformly spreads the applied fluid radially throughout the layer.

Useful particulate spreading layers, including bead spreading layers are disclosed in U.S. Patents 4,670,381; 4,258,001 and 4,430,436. Particularly useful spreading layers are those having a particulate structure formed by organo-polymeric particles and a polymeric adhesive for those particles described in U.S. Patent 4,258,001. The organo-polymeric particles useful in the spreading layer are generally heat-stable, spherical beads having a particle size in the range of from 20 to 40 μm in diameter or even smaller.

The particles can be composed of a wide variety of organic polymers, including both natural and synthetic polymers, having the requisite properties. Preferably, however, they are composed of one or more addition polymers described in the aforementioned patents.

The particulate layer of the element is carried on a suitable support. Such a support can be any suitable dimensionally stable, and preferably, nonporous and transparent (i.e. radiation transmissive) material which transmits electromagnetic radiation of a wavelength between 200 and 900 nm. A support of choice for a particular element should be compatible with the intended mode of detection (reflection, transmission or fluorescence spectroscopy). Useful support materials include polystyrene, polyesters [for example poly(ethylene terephthalate)], polycarbonates, cellulose esters (for example cellulose acetate), and so forth

The element can comprise one or more additional layers, for example separate or a combined reagent/spreading layer and a gelatin/buffer layer containing other necessary additives, coupling enzymes, and so forth

The gelatin/buffer layer or the reagent layer or the spreading layer of the element can contain the indicator composition comprising one or more reagents dispersed in one or more synthetic or natural binder materials, such as gelatin, or other naturally-occurring colloids, homopolymers and copolymers, such as poly(acrylamide), poly(vinylpyrrolidone), poly(N-isopropylacrylamide), poly(acrylamide-co-N-vinyl-2-pyrrolidone) and similar copolymers.

Other optional layers, for example subbing layers, radiation-blocking layers, and so forth can be included if desired. All layers of the element are in fluid contact with each other, meaning that fluids and reagents and uncomplexed reaction products in the fluids can pass between superposed regions of adjacent layers.

The layers of the element can contain a variety of other desirable but optional components, including surfactants, thickeners, buffers, hardeners, antioxidants, coupler solvents, and other materials known in the art. The amounts of

these components are also within the skill of a worker in the art.

The elements can be used to determine low concentrations of immunologically reactive ligands in a liquid, such as a biological fluid (for example, whole blood, serum, plasma, urine, spinal fluid, suspensions of human or animal tissue, feces, saliva, lymphatic fluid and the like). The ligands can be determined at concentrations as low as $10^{-15}$ molar, and most generally at a concentration of from $10^{-10}$ to $10^{-4}$ molar.

Ligands which can be so determined, either quantitatively or qualitatively, include therapeutic drugs (for example, phenobarbital, theophylline, gentamicin, quinidine, phenytoin, propanolol, carbamazepine, tobramycin, lidocaine, procainamide and the like), natural or synthetic steroids (for example, cortisol, aldosterone, testosterone, progesterone, estriol, and so forth), hormones (for example, thyroid hormones, peptide hormones, insulin, and so forth), proteins (for example albumin, IgG, IgM, ferritin, C-reactive protein, isoenzymes, apolipoproteins, and so forth), antigens, antibodies including monoclonal antibodies, and other species which will naturally react with a receptor. This invention is particularly useful for the determination of therapeutic drugs, such as digoxin, phenytoin, theophylline, or phenobarbital and hormones such as thyroxine or triiodothyronine.

The assay can be carried out using any enzyme label which can be attached to the ligand to form a labeled ligand. Enzymes, such as glucose oxidase, peroxidases such as horseradish peroxidase (HRP), alkaline phosphatase and galactosidase are preferred labels.

The substrate for the enzyme is present in the element or added thereto as a substrate solution. The substrate can be added to the element prior to or simultaneously with the liquid sample, or after completion of the binding reaction. It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. For example, if the enzyme label is a peroxidase, the substrate is hydrogen peroxide. Using glucose oxidase as an example, the substrate glucose is generally present in the reagent layer or added as a substrate solution to yield 0.01 moles/$m^2$, and preferably from 0.001 to 0.1 mole/$m^2$. A worker skilled in the art would know how to adjust the amount of a particular substrate for the amount of enzyme label used in the assay.

The reagent layer may contain an indicator composition comprising one or more reagents which provide a detectable species as a result of the reaction catalyzed by the label. Preferably, the indicator composition is a colorimetric indicator composition which provides a colorimetrically detectable species as a result of enzymatic reaction of an enzyme-labeled ligand analog with a substrate.

The indicator composition can be a single compound which produces a detectable dye upon enzymatic reaction, or a combination of reagents which produce the dye. For example, when glucose is used as the substrate and glucose oxidase as the enzyme label, the colorimetric indicator composition can include a coupler and an oxidizable compound which react to provide a dye. Alternatively, the composition can include a leuco dye and peroxidase or another suitable peroxidative compound which generates a detectable dye as a result of the formation of hydrogen peroxide produced when glucose oxidase converts glucose to gluconic acid. Useful leuco dyes are known in the art and include those, for example, described in U.S. Patent 4,089,747 (issued May 16, 1978 to Bruschi) and U.S. Serial No. 612,509, filed May 21, 1984 by Babb and others. The particular amounts of the colorimetric indicator composition and its various components are within the skill of a worker in the art.

The labeled ligands can be prepared using known starting materials and procedures, or obtained commercially. Generally, the ligand is attached to the label (for example an enzyme moiety) through a covalent bond.

The immunoassay can be manual or automated. In general, the amount of a ligand in a liquid is determined by taking the element from a supply roll, chip packet or other source and physically contacting a finite area of the spreading layer with a sample of the liquid, for example 1 to 100 µL. The finite area which is contacted is generally no more than 100 $mm^2$.

The amount of ligand is determined by passing the element through a suitable apparatus for detecting the complexed ligand analog directly or the detectable species formed as a result of enzymatic reaction of an enzyme label and a substrate. For example, the species can be detected with suitable or spectrophotometric apparatus using generally known procedures. In an enzymatic reaction, the resulting product is determined by measuring, for example, the rate of change of reflection or transmission density in the finite area which was contacted with the test sample. The area which is measured is generally from 3 to 5 mm. The amount of ligand in the liquid sample is inversely proportional to the amount of label measured in the finite area. Generally, label measurement is made 5 to 180 seconds after sample contact and spreading or application of a substrate solution.

A typical element of this invention is presented below. It will be understood by those skilled in the art that the principle of the present invention can be usefully incorporated into any immunoassay element.

Immunoassay Element of the Invention

| | | Dry Coverage (g/M²) |
|---|---|---|
| Labeled Ligand Coating | Phenytoin-HRP | .000016 |
| | MOPS, pH 7.0 | .0045 |
| | Bovine serum albumin | .000215 |
| | A-100 | .00108 |
| | 4-Hydroxyacetanillde | .000325 |
| Bead Spreading Layer (BSL) | TES, pH 7.0 | .219 |
| | Dimedone | .05 |
| | Triarylimidazole leuco dye | .2 |
| | Dimethylsulfoxide | 1.8 |
| | MaWnaMt | 2.583 |
| | VtE Beads | 130 |
| | Zonyl FSN | .057 |
| | Methanol | .675 |
| | Potassium phosphate, pH 7.0 | .039 |
| | antiphenytoin-SUb beads | 0.1-0.2 |
| Gelatin Layer | *Rousseleau type iv gelatin* | 10 |
| | 4-Hydroxyacetanilide | .15 |
| | TES, pH 7 0 | 4.58 |
| | TX-100 | .02 |
| | BVSME (hardener) | .15 |

The above element can be used to quantify ligands other than phenytoin stated hereinbefore. The selected labeled ligand depends upon the particular assay. Only one such labeled ligand is used in a single assay with a paired antibody in the bead spreading layer so that a specific antibody-ligand pair exists for each assay.

The names and symbols used in the above element have the following meanings:

| | |
|---|---|
| MOPS: | 3-Morpholinopropanesulfonic acid buffer |
| Dimedone: | 5,5-Dimethyl-1,3-cyclohexanedione |
| Triarylimidazole leuco dye: | 4,5-Bis(4-dimethylaminophenyl)-2-(4-hydroxy-3,5-dimethoxyphenyl)imidazole blue-forming leuco dye. |
| MaWnaMt: | Poly(methyl acrylate-co-sodium-2-acrylamido-2-methylpropanesulfonate-co-2-acetoacetoxyethyl methacrylate). |
| VtE: | Poly(m-&p-vinyltoluene-co-methacrylic acid). |
| Zonyl FSN: | A nonionic, fluorinated surfactant sold by E. I. du Pont de Nemours. |
| SUb: | Poly[styrene-co-m-&p-(2-chloroethylsulfonylmethyl)styrene]. |

| | |
|---|---|
| Antiphenytoin-SUb beads: | Particles of SUb polymer having the appropriate immunoreactive antibody covalently bound thereto. |
| TX-100: | Triton® X-100, an octylphenoxy polyethoxy ethanol nonionic surfactant sold by Rohm and Haas. |
| HRP: | Horseradish peroxidase |
| BVSME: | Bis(vinylsulfonylmethyl)ether gelatin hardener. |
| Rousseleau Type IV Gel: | Bone gelatin. |

The gelatin layer was coated on a subbed, gelatin washed poly(ethylene terephthalate) support, and the remaining layers were coated on top. The bead spreading layer was coated over the gelatin layer. All of the aforementioned layers were coated using conventional coating techniques known in the art for making dry immunoassay elements mentioned, *supra.* The labeled ligand was gravure coated according to the procedure presented, *supra.*

The following comparative and invention examples demonstrate the operability of the present invention. The immunoassay element used was the same for all examples except for the difference in coating procedures and antibody in example 6.

In the examples the assay was carried out step-wise according to the following protocol. Ten µL of a sample was spotted on the top surface of a dry immunoassay element of the invention. The element with the now spotted sample was then incubated at 37°C for 5 minutes. It is expected that with the elements of this invention equilibrium in the competition between labeled and unlabeled ligand for receptor binding sites in the bead spreading layer will be complete within 20, preferably within 5 minutes. After this period of incubation the element was removed from the incubator and contacted with 10 µL of enzyme substrate solution. For the assays used in the examples the label is horseradish peroxidase (HRP) and the substrate solution is 0.3% by weight $H_2O_2$. The substrate solution also contains sodium phosphate buffer (pH 6.8) 0.01M, 4'-hydroxyacetanilide 0.005M, diethylenetriaminepentaacetic acid 10 µM and a surfactant. Bound HRP-labeled ligand catalyzes the oxidation of a colorless leuco dye to its colored form. Such dyes are well known in the dry analytical element art and will not be described in detail here. In the examples presented herein the dye is a triarylimidazole leuco dye. The rate of the catalyzed reaction is measured from the change in reflection density over time at 37°C. Methods and means for measuring reflection density are well know in the analytical arts. The reflection density is converted to transmission density using the Clapper-Williams transform.

In each of the following examples a comparison was made between the dose response curves of assays carried out with elements of the invention and with the same elements that did not have a coated labeled ligand to determine the extent of prebinding. With the latter elements the labeled ligand is added to the 10 µL sample and spotted on the element concurrently with the sample.

Comparative examples 1-4 are presented below to illustrate the high level of receptor/labeled ligand prebinding that occurred with a number of unsuccessful coating processes. Several unsuccessful approaches were considered and attempted for coating the labeled ligand in a dry analytical element in an attempt to avoid prebinding and adverse effects on receptors such as antibodies. Unsuccessful approaches studied to achieve the present invention include direct addition (comparative example 1) of phenytoin-HRP to the coating composition used to form the particulate layer containing polymeric beads (referred to hereinafter as a bead spreading layer or BSL) and immobilized antibodies; incorporation of phenytoin-HRP into the coating composition for the bead spreading layer by a mix melting procedure (comparative example 2); use of a Dual X-Hopper to coat an aqueous phenytoin-HRP solution from the top X-slot and the bead spreading layer containing antibody-beads for phenytoin from the bottom X-slot; and coating the phenytoin-HRP in an unhardened gelatin layer under the bead spreading layer which contained immobilized antibodies for phenytoin. Comparative examples 1-4, infra, illustrate the level of prebinding that occurs with these coating techniques thereby showing that a process for successfully coating a labeled ligand in an element directly over a bead spreading layer containing receptors for the ligand substanially free of prebinding is unobvious.

<u>Comparative Example 1:</u> Direct Addition of Phenytoin-HRP to the Bead Spreading Layer Coating Composition Containing Antibody-Beads for Phenytoin

Antibodies to phenytoin which were covalently attached to approximately 1µ beads and a solution of phenytoin-HRP were both added to standard bead spreading layer coating composition. The coating composition was held about 30 minutes and then coated over a hardened gelatin layer of the type known from the aforementioned U.S. Patent 4,258,001 containing 0.5M phosphate buffer at pH 7.0. A control element was prepared exactly as described except phenytoin-HRP was excluded from the bead spreading layer thereof. The comparative test element of this example was tested by spotting the sample on the bead spreading layer thereof. The control element was tested by adding phenytoin-HRP to the sample containing phenytoin prior to spotting the sample on the control bead spreading layer. Both elements were tested according to the previously described assay protocol using a series of aqueous phenytoin solutions ranging from $10^{-9}$ to $10^{-4}$ M containing either none or 1 nM of phenytoin-HRP . The rate results are summarized

in Table I and a plot of that data is shown in figure 1.

TABLE I

| Coating | Phenytoin Conc., M | Rate DT/Min (40-70 sec) | Rate Range ($10^{-4}$-$10^{-9}$ M) |
|---|---|---|---|
| Control | 1 x $10^{-4}$ | .0078 | .0458 |
| | 1 x $10^{-5}$ | .0112 | |
| | 1 x $10^{-6}$ | .0291 | |
| | 1 x $10^{-7}$ | .0484 | |
| | 1 x $10^{-8}$ | .0525 | |
| | 1 x $10^{-9}$ | .0536 | |
| Coated Phenytoin-HRP | 1 x $10^{-4}$ | .0296 | .0114 |
| | 1 x $10^{-5}$ | .0316 | |
| | 1 x $10^{-6}$ | .0351 | |
| | 1 x $10^{-7}$ | .0396 | |
| | 1 x $10^{-8}$ | .0403 | |
| | 1 x $10^{-9}$ | .041 | |

When the phenytoin-HRP was coated even very high phenytoin concentrations ($10^{-4}$ M) produced only a small change in observed rate compared to the results with the control in which the label was absent from the coating but added to the sample. The results indicate that a significant amount of prebinding occurred when the label was coated in the bead spreading layer.

<u>Comparative Example 2:</u> Incorporation of Phenytoin-HRP By A Mix-Melting Procedure Into The Bead Spreading Layer Coating Composition Containing Antibody-Beads For Phenytoin

A standard aqueous bead spreading layer coating composition was triple mix-melted a few feet from the coating hopper with antibody-beads and a separate phenytoin-HRP solution. The coatings were mixed in a ratio of 25 parts of beads:1 part antibody-beads:1 part of phenytoin-HRP. The latter composition was coated over a hardened gel layer as in comparative example 1 to form the element of this example. The control element of example 1 was used as the control for this example. Both elements were tested as described in comparative example 1. The rate results are summarized in Table II. A plot of that data is presented in figure 2.

TABLE II

| Coating | Phenytoin Conc., M | Rate DT/Min (40-70 sec) | Rate Range ($10^{-4}$-$10^{-9}$ M) |
|---|---|---|---|
| Control | 1 x $10^{-4}$ | .0078 | .0458 |
| | 1 x $10^{-5}$ | .0112 | |
| | 1 x $10^{-6}$ | .0291 | |
| | 1 x $10^{-7}$ | .0484 | |
| | 1 x $10^{-8}$ | .0525 | |
| | 1 x $10^{-9}$ | .0536 | |
| Coated Phenytoin-HRP | 1 x $10^{-4}$ | .0272 | .0113 |
| | 1 x $10^{-5}$ | .0282 | |
| | 1 x $10^{-6}$ | .0324 | |
| | 1 x $10^{-7}$ | .0379 | |
| | 1 x $10^{-8}$ | .0384 | |
| | 1 x $10^{-9}$ | .0385 | |

The results indicate that a significant amount of prebinding has occurred when the labeled phenytoin was coated from a coating composition prepared as in example 2.

Comparative Example 3: Use of a Dual X-Hopper to Coat an Aqueous Phenytoin-HRP Coating Composition From the Top X-Slot and the Bead Spreading Layer Coating containing Antibody-Beads For Phenytoin From The Bottom X-Slot

A bead spreading layer coating composition as prepared in comparative example 1 containing phenytoin antibody-beads was coated from the bottom X-slot of a dual X-Hopper and an aqueous phenytoin-HRP coating composition was coated from the top X-slot. The coated ratio was 12.5 parts bead spreading layer coating composition to 1 part of phenytoin-HRP coating composition. The bead spreading layer coating composition was coated over a hardened gelatin layer as previously described. Two control elements were prepared by coating the bead spreading layer coating composition from the bottom X-slot and 1) nothing from the top X-slot or (2) coating the phenytoin-HRP coating composition (without the labeled phenytoin) from the top X-slot. The control elements and the element containing the coated phenytoin-HRP were tested as in comparative example 1. The rate results are summarized in table III. A plot of these rate results are presented in figure 3.

TABLE III

| Coating | Phenytoin Conc., M | Rate DT/Min (40-70 sec) | Rate Range ($10^{-4}$-$10^{-9}$ M) |
|---|---|---|---|
| Control 1 | $1 \times 10^{-4}$ | .0049 | .0772 |
| | $1 \times 10^{-5}$ | .0070 | |
| | $1 \times 10^{-6}$ | .0378 | |
| | $1 \times 10^{-7}$ | .0730 | |
| | $1 \times 10^{-8}$ | .0803 | |
| | $1 \times 10^{-9}$ | .0821 | |
| Control 2 | $1 \times 10^{-4}$ | .0043 | .0752 |
| | $1 \times 10^{-5}$ | .0058 | |
| | $1 \times 10^{-6}$ | .0379 | |
| | $1 \times 10^{-7}$ | .0709 | |
| | $1 \times 10^{-8}$ | .0758 | |
| | $1 \times 10^{-9}$ | .0795 | |
| Dual | $1 \times 10^{-4}$ | .0472 | .0330 |
| X-Hopper | $1 \times 10^{-5}$ | .0506 | |
| Coated | $1 \times 10^{-6}$ | .0575 | |
| Phenytoin- | $1 \times 10^{-7}$ | .0718 | |
| HRP | $1 \times 10^{-8}$ | .0792 | |
| | $1 \times 10^{-9}$ | .0802 | |

The results indicate that a significant amount of prebinding has occurred.

Comparative Example 4: Coating the Phenytoin-HRP In an unhardened Gelatin Layer Under the Bead Spreading Layer which Contains Antibody-Beads for Phenytoin

This element was prepared as follows. Unhardened gelatin was coated with phenytoin-HRP over a hardened gelatin layer as described in comparative example 1 except that 0.05M TES buffer was used at a pH of 7.0. A bead spreading layer coating composition containing antibody-beads as described in comparative example 1 was coated over the unhardened gelatin layer containing the phenytoin-HRP. Two control elements were prepared by 1) coating the bead spreading layer coating composition with the antibody-beads over the hardened gelatin layer directly without an intervening unhardened gelatin layer and 2) by coating the bead spreading layer coating composition with antibody-beads over the unhardened gelatin layer (without phenytoin-HRP). Each of the three elements was tested as described in comparative example 1. The rate results are summarized in Table IV. A plot of the data is presented in Figure 4.

TABLE IV

| Coating | Phenytoin Conc., M | Rate DT/Min (40-70 sec) | Rate Range ($10^{-4}$-$10^{-9}$ M) |
|---|---|---|---|
| Control 1 | $1 \times 10^{-4}$ | .0026 | .0662 |
| | $1 \times 10^{-5}$ | .0069 | |
| | $1 \times 10^{-6}$ | .0309 | |
| | $1 \times 10^{-7}$ | .0600 | |
| | $1 \times 10^{-8}$ | .0666 | |
| | $1 \times 10^{-9}$ | .0688 | |
| Control 2 | $1 \times 10^{-4}$ | .0059 | .0446 |
| | $1 \times 10^{-5}$ | .0060 | |
| | $1 \times 10^{-6}$ | .0264 | |
| | $1 \times 10^{-7}$ | .0467 | |
| | $1 \times 10^{-8}$ | .0496 | |
| | $1 \times 10^{-9}$ | .0505 | |
| Coated Phenytoin-HRP | $1 \times 10^{-4}$ | .0219 | .0125 |
| | $1 \times 10^{-5}$ | .0197 | |
| | $1 \times 10^{-6}$ | .0247 | |
| | $1 \times 10^{-7}$ | .0338 | |
| | $1 \times 10^{-8}$ | .0343 | |
| | $1 \times 10^{-9}$ | .0344 | |

The results indicate that significant prebinding has occurred when the phenytoin-HRP was coated in this manner.

The following examples demonstrate the coating procedure used to make the elements of the present invention having a coating of a labeled ligand. Although these examples involve the use of phenytoin-HRP , it will be clear to those skilled in the art that elements having other labeled ligands can also be made by following the teachings herein.

Example 5: Gravure Coating of an Aqueous Phenytoin-HRP Solution over a Preformed Bead Spreading Layer Containing Antibody-Beads for Phenytoin

An element within the scope of the present invention was prepared by using the gravure process previously described. A phenytoin-HRP coating composition was coated over a preformed bead spreading layer containing phenytoin antibody-beads. The bead layer was coated over a hardened gelatin layer as previously described. A control element was similarly prepared except that the coated phenytoin-HRP was omitted. Both the example element and the control element were tested as in comparative example 1 except that the test sample applied to the control element included the phenytoin-HRP. The rate results are summarized in Table V and a plot of the data is shown in Figure 5.

TABLE V

| Coating | Phenytoin Conc., M | Rate DT/Min (40-70 sec) | Rate Range ($10^{-4}$-$10^{-9}$ M) |
|---|---|---|---|
| Control | $1 \times 10^{-4}$ | .0078 | .0759 |
| | $1 \times 10^{-5}$ | .0179 | |
| | $1 \times 10^{-6}$ | .0435 | |
| | $1 \times 10^{-7}$ | .0775 | |
| | $1 \times 10^{-8}$ | .0820 | |
| | $1 \times 10^{-9}$ | .0837 | |
| Gravure Coated Phenytoin-HRP | $1 \times 10^{-4}$ | .0113 | .0609 |
| | $1 \times 10^{-5}$ | .0177 | |
| | $1 \times 10^{-6}$ | .0444 | |
| | $1 \times 10^{-7}$ | .0579 | |
| | $1 \times 10^{-8}$ | .0700 | |
| | $1 \times 10^{-9}$ | .0722 | |

The results indicate that very little prebinding occurred when the phenytoin-HRP was coated using the described gravure coating procedure. The rate range is about 80% of that obtained with spotted phenytoin-HRP when this antibody and labeled ligand combination are used for the phenytoin assay.

Example 6: This example of an element of the invention was prepared and tested against a control as in example 5 except that a different antibody for phenytoin was used. Also the bead spreading layer coating composition was coated over a hardened gelatin containing 0.2 M TES buffer at pH 7.0. A control coating was used for testing with spotted phenytoin-HRP which did not go through the gravure process. The elements were tested as in comparative example 1 using serum-based calibrators containing either none or 1 nM phenytoin-HRP . The rate results are summarized in Table VI and a plot of the data is shown in Figure 6.

TABLE VI

| Coating | Phenytoin Conc., µg/mL | Rate DT/Min (40-100 sec) | Rate Range (0-64 µg/mL) |
|---|---|---|---|
| Control | 0 | .1032 | .0790 |
| | .5 | .0972 | |
| | 1 | .0940 | |
| | 2 | .0892 | |
| | 4 | .0792 | |
| | 8 | .0665 | |
| | 16 | .0492 | |
| | 32 | .0366 | |
| | 64 | .0242 | |
| Gravure Coated Phenytoin-HRP | 0 | .0934 | .0660 |
| | .5 | .0949 | |
| | 1 | .0908 | |
| | 2 | .0849 | |
| | 4 | .0750 | |
| | 8 | .0633 | |
| | 16 | .0514 | |
| | 32 | .0379 | |
| | 64 | .0274 | |

The results indicate that very little prebinding occurred when the phenytoin-HRP was coated as described. The rate range is about 84% of that obtained with spotted phenytoin-HRP when this antibody and labeled ligand combination are used for the phenytoin assay.

## Claims

1. A dry immunoassay analytical element, for assaying a ligand, comprising (a) a particulate layer containing a fixed concentration of immobilized receptor for the ligand, and (b) a coating containing an enzyme labelled ligand at a coverage within a predetermined range gravure-coated directly over the particulate layer (a), without an intervening barrier layer, wherein the element is substantially free of binding reactions between the receptor and the labelled ligand.

2. The element of claim 1 wherein the labelled ligand is present at a coverage of 4 to 64 $\mu g/m^2$.

3. The element of claim 1 or claim 2 wherein the particulate layer is a bead spreading layer in which the receptors are immobilised on beads.

4. The element of any one of claims 1 to 3 wherein the ligand comprises a hapten selected from digoxin, phenytoin,

carbamazepine, phenobarbital, C-reactive protein, thyroxine and theophylline.

**5.** The element of any one of claims 1 to 4 wherein the receptor is an antibody for the ligand.

**6.** The element of any one of claims 1 to 5, further including at least one additional layer comprising at least a portion of an indicator composition which produces a detectable change in the element that allows quantitative measurement of the ligand.

**7.** The element of claim 6 wherein the additional layer comprises a buffer and an electron transfer agent.

**8.** The element of claim 6 or 7 wherein the additional layer comprises a dye precursor which is converted into a dye by other portions of the indicator composition.

**9.** A method of making a dry immunoassay analytical element comprising the steps of:

A. providing a dry immunoassay analytical element having a particulate layer containing a fixed concentration of immobilized receptor for the ligand;
B. determining the concentration of labelled ligand needed to achieve acceptable immunoassay performance when the immunoassay is performed by spotting the analytical element with the labelled ligand concurrently with a sample;
C. empirically determining the level of coated labelled ligand coverage needed with the same analytical element to achieve acceptable assay performance by:

(i) gravure-coating, directly over the particulate receptor layer of the element used to establish optimum spotted labelled ligand levels, a coating containing an enzyme labelled ligand at a predetermined coverage;
(ii) conducting a series of assays with test samples containing a known concentration of the ligand;
(iii) comparing the results of the assays with the known concentration of ligand;
(iv) repeating steps (i) and (ii) as needed, varying the labelled ligand coverage according to the results seen in step (iii), to determine the labelled ligand coverage required; and

D. gravure-coating the coverage level of the labelled ligand empirically determined in step C.

**10.** The method of claim 9, wherein the labelled ligand is present at a coverage of 4 to 64 ug/m$^2$.

**11.** The method of claim 9 or claim 10 wherein the labelled ligand coverage is less than, the same as or several multiples greater than the labelled ligand concentration needed when the assay is carried out according to step B.

**12.** A method for the assay of an immunologically reactive ligand in a liquid using the element of any one of claims 1 to 8, comprising the steps of:

A. contacting a finite area of the particulate layer with a sample of the liquid to form (i) an immobilized ligand-receptor complex and (ii) an immobilized labelled ligand-receptor complex within the finite area;
B. contacting the finite area of the particulate layer with a substrate solution; and
C. determining the concentration of ligand.

**Patentansprüche**

**1.** Trockenes, analytisches Immunoassay-Element zum Testen eines Liganden, umfassend (a) eine teilchenförmige Schicht, die eine feststehende Konzentration eines immobilisierten Rezeptors für den Liganden enthält, und (b) einen Überzug, der einen enzymmarkierten Liganden in einer Beschichtungsmenge innerhalb eines vorbestimmten Bereichs enthält und durch Tiefdruck direkt auf die teilchenförmige Schicht (a) ohne eine zwischenliegende Sperrschicht aufgebracht ist, wobei das Element im wesentlichen frei von Bindungsreaktionen zwischen dem Rezeptor und dem markierten Liganden ist.

**2.** Element nach Anspruch 1, wobei der markierte Ligand in einer Beschichtungsmenge von 4 bis 64 $\mu$g/m$^2$ vorhanden ist.

3. Element nach Anspruch 1 oder 2, wobei es sich bei der teilchenförmigen Schicht um eine Perlstreichschicht handelt, bei der die Rezeptoren an Perlen immobilisiert sind.

4. Element nach einem der Ansprüche 1 bis 3, wobei der Ligand ein Hapten umfaßt, das unter Digoxin, Phenytoin, Carbamazepin, Phenobarbital, C-reaktivem Protein, Thyroxin und Theophyllin ausgewählt ist.

5. Element nach einem der Ansprüche 1 bis 4, wobei es sich beim Rezeptor um einen Antikörper für den Liganden handelt.

6. Element nach einem der Ansprüche 1 bis 5, ferner umfassend mindestens eine zusätzliche Schicht, die mindestens einen Teil einer Indikatorzusammensetzung enthält, die eine nachweisbare Veränderung im Element ergibt, die eine quantitative Messung des Liganden ermöglicht.

7. Element nach Anspruch 6, wobei die zusätzliche Schicht einen Puffer und ein Elektronenübertragungsmittel umfaßt.

8. Element nach Anspruch 6 oder 7, wobei die zusätzliche Schicht einen Farbstoffvorläufer umfaßt, der durch andere Teile der Indikatorzusammensetzung in einen Farbstoff übergeführt wird.

9. Verfahren zur Herstellung eines trockenen analytischen Immunoassay-Elements, umfassend folgende Stufen:

   A. Bereitstellen eines trockenen analytischen Immunoassay-Elements mit einer teilchenförmigen Schicht, die eine feststehende Konzentration eines immobilisierten Rezeptors für den Liganden enthält;
   B. Bestimmen der Konzentration an markiertem Liganden, die zur Erzielung eines annehmbaren Immunoassay-Verhaltens erforderlich ist, wenn der Immunoassay durchgeführt wird, indem man auf das analytische Element den markierten Liganden gleichzeitig mit einer Probe aufsetzt;
   C. empirische Bestimmung der Konzentration der Beschichtungsmenge des markierten Liganden, die zusammen mit dem gleichen analytischen Element erforderlich ist, um ein annehmbares Testverfahren zu erreichen, indem man:

      (i) direkt über die teilchenförmige Rezeptorschicht des Elements, das zur Festlegung der optimalen Aufsetzkonzentrationen des markierten Liganden verwendet wird, durch Tiefdruck einen Überzug aufbringt, der einen enzymmarkierten Liganden in einer vorbestimmten Beschichtungsmenge enthält;
      (ii) Durchführen einer Reihe von Tests mit Testproben, die eine bekannte Konzentration des Liganden enthalten;
      (iii) Vergleichen der Ergebnisse der Tests mit der bekannten Ligandenkonzentration;
      (iv) Wiederholung der Stufen (i) und (ii) je nach Bedarf, wobei man die markierte Liganden-Beschichtungsmenge je nach den in Stufe (ii) erzielten Ergebnissen variiert, um die erforderliche Beschichtungsmenge für den markierten Liganden zu bestimmen; und

   D. Aufbringen der in Stufe C empirisch bestimmten Beschichtungsmenge des markierten Liganden durch Tiefdruck.

10. Verfahren nach Anspruch 9, wobei der markierte Ligand in (i) in einer Beschichtungsmenge von 4 bis 64 $\mu g/m^2$ vorhanden ist.

11. Verfahren nach Anspruch 9 oder 10, wobei die Beschichtungsmenge des markierten Liganden im Vergleich zu der Konzentration des markierten Liganden, die bei Durchführung des Tests gemäß Stufe B erforderlich ist, geringer oder gleich groß ist oder ein Mehrfaches davon beträgt.

12. Verfahren zur Bestimmung eines immunologisch reaktiven Liganden in einer Flüssigkeit unter Verwendung des Elements nach einem der Ansprüche 1 bis 8, umfassend folgende Stufen:

   A. Kontaktieren einer begrenzten Fläche der teilchenförmigen Schicht mit einer Probe der Flüssigkeit unter Bildung (i) eines immobilisierten Liganden-Rezeptor-Komplexes und (ii) eines immobilisierten, markierten Liganden-Rezeptor-Komplexes innerhalb des begrenzten Bereichs;
   B. Kontaktieren des begrenzten Bereichs der teilchenförmigen Schicht mit einer Substratlösung; und
   C. Bestimmung der Konzentration des Liganden.

**Revendications**

1. Elément analytique en phase sèche pour essai immunologique, destiné à analyser un ligand, comprenant (a) une couche de globules contenant une concentration fixe de récepteur immobilisé complémentaire au ligand, et (b) un revêtement contenant un ligand à marquage enzymatique suivant un taux de couverture compris dans une gamme prédéterminée, appliqué par gravure directement sur la couche de globules (a), en absence de couche de barrage intercalaire, dans lequel l'élément est essentiellement exempt de réactions de fixation entre le récepteur et le ligand marqué.

2. Elément selon la revendication 1, dans lequel le ligand marqué est présent suivant un taux de couverture de 4 à 64 $\mu g/m^2$.

3. Elément selon la revendication 1 ou la revendication 2, dans lequel la couche de globules est une couche d'étalement en billes dans laquelle les récepteurs sont immobilisés sur des billes.

4. Elément selon l'une quelconque des revendications 1 à 3, dans lequel le ligand comprend un haptène choisi parmi la digoxine, la phénytoïne, la carbamazépine, le phénobarbital, la protéine C-réactive, la thyroxine et la théophylline.

5. Elément selon l'une quelconque des revendications 1 à 4, dans lequel le récepteur est un anticorps dirigé contre le ligand.

6. Elément selon l'une quelconque des revendications 1 à 5, comprenant en outre au moins une couche auxiliaire comprenant au moins une partie d'une composition indicateur qui produit un changement détectable au niveau de l'élément permettant un dosage quantitatif du ligand.

7. Elément selon la revendication 6, dans lequel la couche auxiliaire comprend un tampon et un agent de transfert d'électrons.

8. Elément selon la revendication 6 ou 7, dans lequel la couche auxiliaire comprend un précurseur de colorant qui est converti en colorant par d'autres parties de la composition indicateur.

9. Procédé de fabrication d'un élément analytique en phase sèche pour essai immunologique comprenant les étapes :

   A. d'obtention d'un élément analytique en phase sèche pour essai immunologique ayant une couche de globules contenant une concentration fixe de récepteur immobilisé complémentaire au ligand;
   B. de détermination de la concentration de ligand marqué nécessaire pour atteindre une performance acceptable pour essai immunologique lorsque l'essai immunologique est conduit en appliquant sous forme de taches le ligand marqué à l'élément analytique en même temps qu'un échantillon;
   C. de détermination par voie empirique du taux de couverture du ligand marqué appliqué nécessaire en présence du même élément analytique pour atteindre une performance d'essai acceptable :

      (i) en appliquant par gravure, directement sur la couche de globules recouverts de récepteur de l'élément utilisé pour établir des taux optimaux de ligand marqué appliqué sous forme de taches, un revêtement contenant un ligand à marquage enzymatique selon un taux de couverture prédéterminé;
      (ii) en conduisant une série d'essais avec les échantillons d'analyse contenant une concentration connue du ligand;
      (iii) en comparant les résultats des essais avec la concentration connue de ligand;
      (iv) en réitérant les étapes (i) et (ii) selon le besoin, en prenant soin de faire varier le taux de couverture de ligand marqué en fonction des résultats observés à l'étape (iii), en vue de déterminer le taux de couverture de ligand marqué nécessaire; et

   D. en appliquant par gravure le ligand marqué selon le taux de couverture déterminé par voie empirique à l'étape C.

10. Procédé selon la revendication 9, dans lequel le ligand marqué est présent suivant un taux de couverture de 4 à 64 $\mu g/m^2$.

**11.** Procédé selon la revendication 9 ou la revendication 10, dans lequel le taux de couverture de ligand marqué est inférieur à, égale à ou est un multiple plus ou moins grand de la concentration de ligand marqué indispensable lorsque l'essai est réalisé selon l'étape B.

**12.** Procédé pour essai d'un ligand immunoréactif dans un liquide utilisant l'élément de l'une quelconque des revendications 1 à 8, comprenant les étapes :

A. de mise en contact d'une surface délimitée de la couche de globules avec un échantillon de liquide en vue de former (i) un complexe ligand-récepteur immobilisé et (ii) un complexe ligand marqué-récepteur immobilisé à l'intérieur de la surface délimitée:
B. de mise en contact de la surface délimitée de la couche de globules avec une solution de substrat; et
C. de détermination de la concentration de ligand.

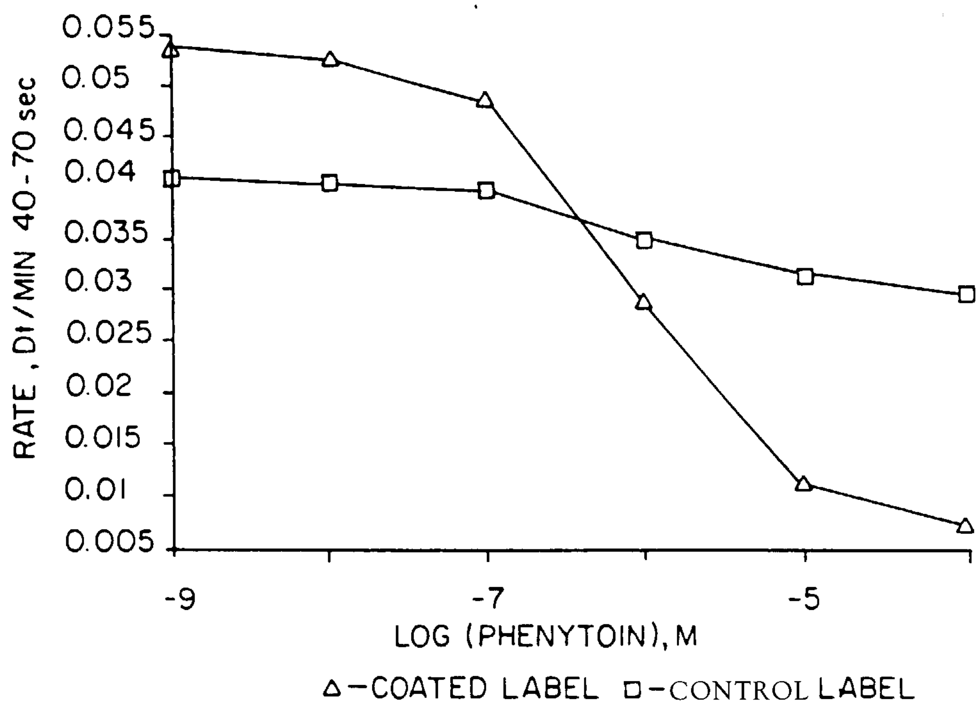
0.055
0.05
0.045
0.04
0.035
0.03
0.025
0.02
0.015
0.01
0.005
RATE, D1/MIN 40-70 sec
-9
-7
-5
LOG (PHENYTOIN), M
Δ-COATED LABEL □-CONTROL LABEL

FIG. 1

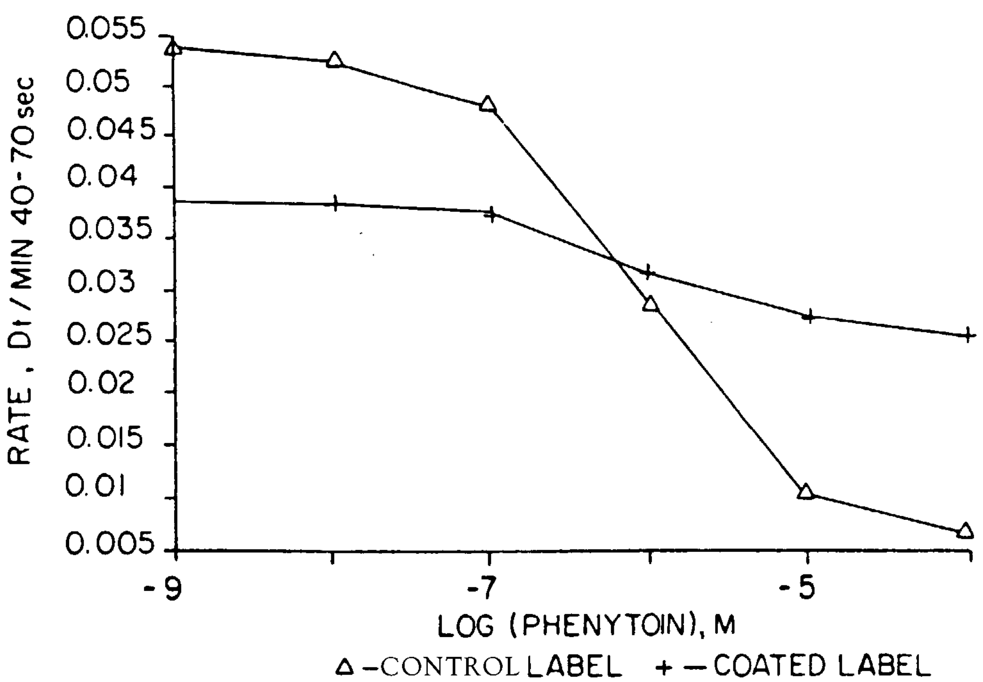
0.055
0.05
0.045
0.04
0.035
0.03
0.025
0.02
0.015
0.01
0.005
RATE, D1/MIN 40-70 sec
-9
-7
-5
LOG (PHENYTOIN), M
Δ-CONTROL LABEL +-COATED LABEL

FIG. 2

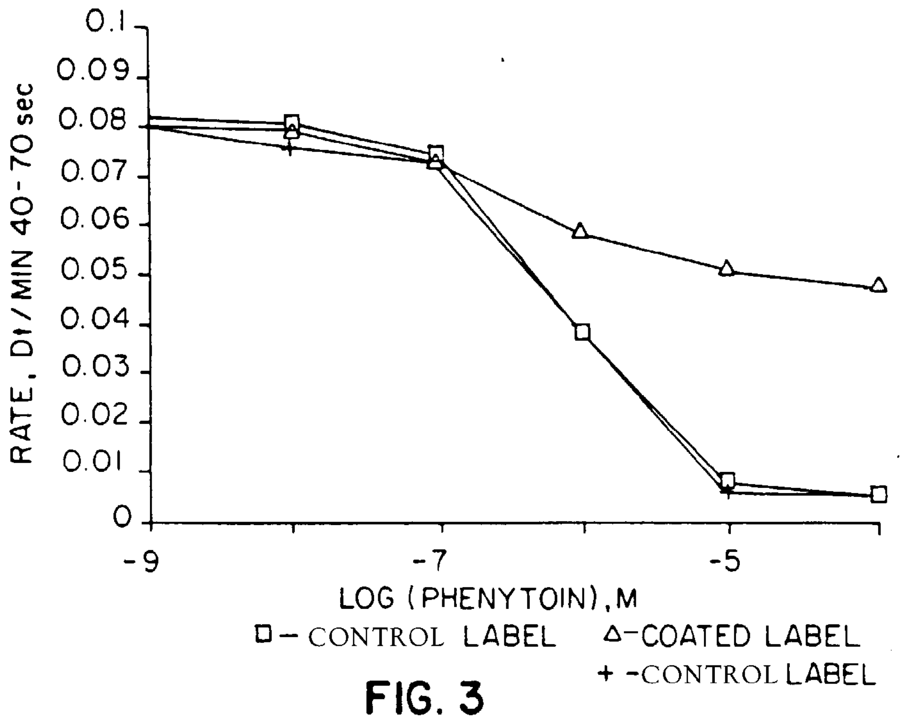
0.1
0.09
0.08
0.07
0.06
0.05
0.04
0.03
0.02
0.01
0
RATE, Dt/MIN 40-70 sec
-9
-7
-5
LOG (PHENYTOIN), M
□ – CONTROL LABEL
Δ–COATED LABEL
+ -CONTROL LABEL

FIG. 3

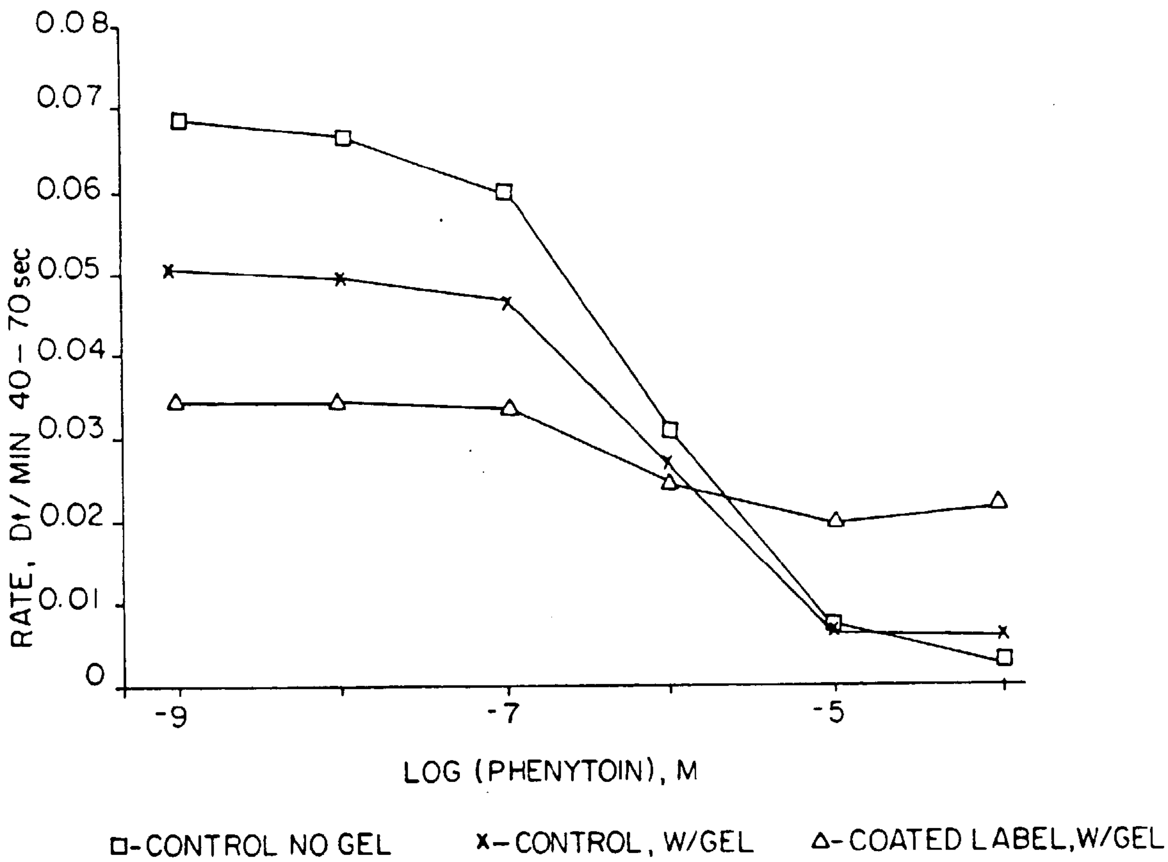
0.08
0.07
0.06
0.05
0.04
0.03
0.02
0.01
0
RATE, Dt/MIN 40 – 70 sec
-9
-7
-5
LOG (PHENYTOIN), M
□-CONTROL NO GEL
x–CONTROL, W/GEL
Δ-COATED LABEL,W/GEL

FIG. 4

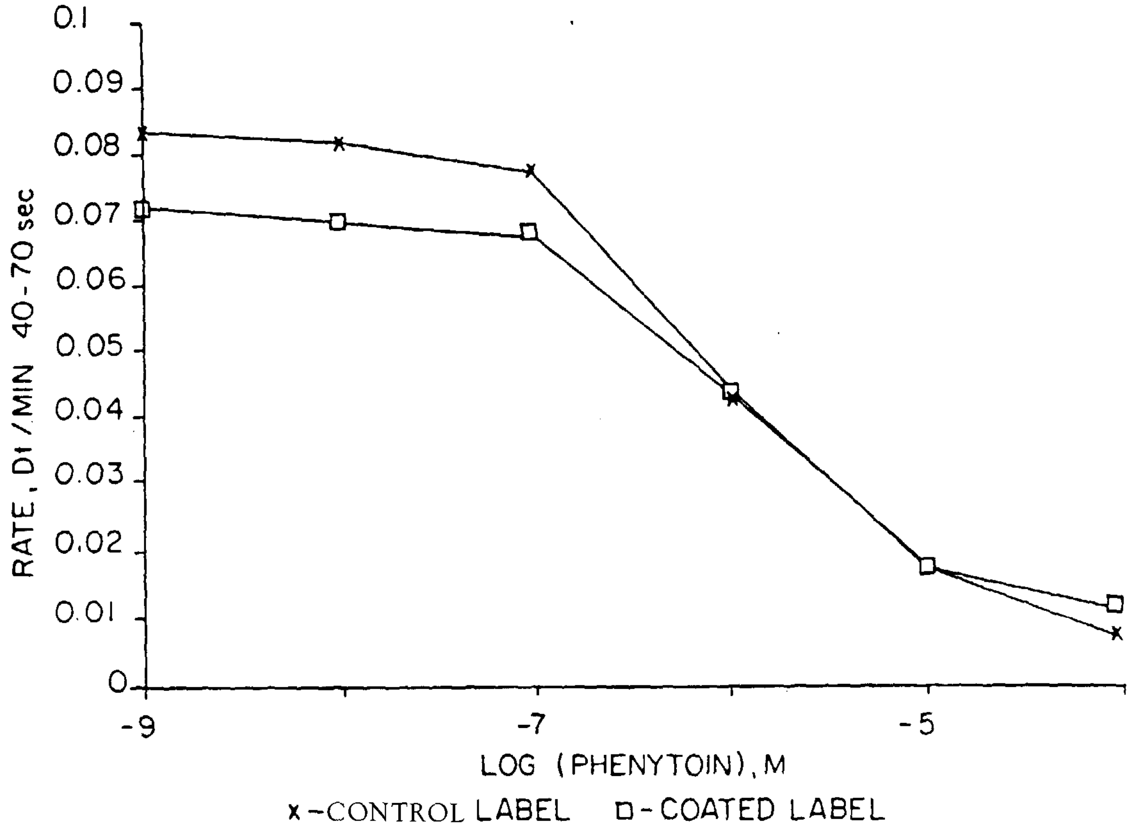

0.1
0.09
0.08
0.07
0.06
0.05
0.04
0.03
0.02
0.01
0
RATE, DI / MIN 40-70 sec
-9
-7
-5
LOG (PHENYTOIN), M
x - CONTROL LABEL
□ - COATED LABEL


FIG. 5

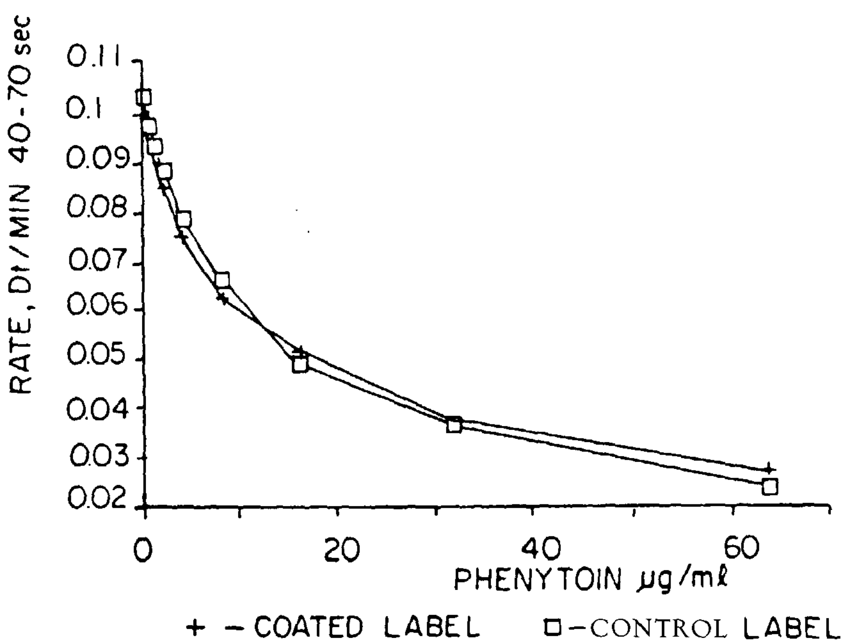

0.11
0.1
0.09
0.08
0.07
0.06
0.05
0.04
0.03
0.02
RATE, DI / MIN 40-70 sec
0
20
40
60
PHENYTOIN μg/mℓ
+ - COATED LABEL
□ - CONTROL LABEL


FIG. 6